(19) Europäisches Patentamt
European Patent Office
Office européen des brevets

(11) Veröffentlichungsnummer : **0 134 934**
**B1**

(12) # EUROPÄISCHE PATENTSCHRIFT

(45) Veröffentlichungstag der Patentschrift : **04.03.87**

(51) Int. Cl.$^4$ : **C 07 C143/72**

(21) Anmeldenummer : **84107338.0**

(22) Anmeldetag : **26.06.84**

(54) **2-Ketosulfonamide und Verfahren zu deren Herstellung.**

(30) Priorität : **30.06.83 DE 3323510**

(43) Veröffentlichungstag der Anmeldung : **27.03.85 Patentblatt 85/13**

(45) Bekanntmachung des Hinweises auf die Patenterteilung : **04.03.87 Patentblatt 87/10**

(84) Benannte Vertragsstaaten : **BE CH DE FR GB IT LI NL**

(56) Entgegenhaltungen :
**EP-B- 0 001 051**
**DE-A- 3 116 129**
**CHEMICAL & PHARMACEUTICAL BULLETIN, Band 21, 1973, Pharmaceutical Society of Japan, Tokyo, HIROAKI YANAGISAWA et al. "Studies on Chemoatherapeutic Agents I.", Seiten 1080-1089**
**THE JOURNAL OF THE AMERICAN CHEMICAL SOCIETY, Band 75, 1953, Easton PA., Mack Printing Company, W.E. TRUCE et al. "The preparation of beta-Ketosulfonyl Chlorides", Seite 2525**

(73) Patentinhaber : **HOECHST AKTIENGESELLSCHAFT**
**Postfach 80 03 20**
**D-6230 Frankfurt am Main 80 (DE)**

(72) Erfinder : **Schmidt, Erwin, Dr.**
**Am Flachsland 26**
**D-6233 Kelkheim (Taunus) (DE)**
Erfinder : **Günther, Dieter, Dr.**
**Nachtigallenweg 1a**
**D-6233 Kelkheim (Taunus) (DE)**
Erfinder : **Kampe, Klaus-Dieter, Dr.**
**Am Rehsteig 1**
**D-6232 Bad Soden am Taunus (DE)**

**EP 0 134 934 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

2-Ketosulfonamide sind wertvolle Zwischenprodukte. Sie dienen als Ausgangsprodukte für Pigmente (vgl. Deutsche Offenlegungsschrift 31 16 129) oder Chemotherapeutika (vgl. Chem. Pharm. Bull. 21 (1973) 5, 1080-1089). Acetonsulfonamid konnte bisher nur durch Oxidation des 2-Hydroxy-propan-sulfonamids hergestellt werden (vgl. Deutsche Offenlegungsschrift 31 16 129). Dieses Vorprodukt ist jedoch nur über das schwer herstellbare instabile 2-Hydroxy-propan-sulfonsäurechlorid zugänglich. Am Stickstoff substituierte Acetonsulfonamide können dagegen auf diesem Wege nicht hergestellt werden, was die enge Begrenzung des genannten Verfahrens zeigt.

Bei einem zweiten Verfahren zur Herstellung von 2-Ketosulfonamiden, nach dem sich insbesondere aromatische 2-Ketosulfonamide herstellen lassen, verwendet man als Ausgangspunkt ein geeignetes Acetophenon.

Dieses wird mit Schwefeltrioxid in die entsprechende Sulfonsäure überführt. Nach der Umsetzung mit Phosphorchloriden (z. B. Phosphortrichlorid, Phosphorpentachlorid) zum Sulfonsäurechlorid wird dieses mit Aminen zum Sulfonamid umgesetzt (vgl. J. Amer. Chem. Soc. *75*, 2525 (1953)).

Die Verwendung solch reaktiver Verbindungen wie Schwefeltrioxid oder der Phosphorchloride führt jedoch zu Nebenreaktionen, die eine aufwendige Reinigung der Zwischenprodukte erfordern.

Infolge der Mängel der beiden Verfahren zur Herstellung von 2-Ketosulfonamiden bestand die Aufgabe, eine einfachere, breiter anwendbare Methode zur Herstellung dieser Verbindungen zu finden.

Es wurde nun ein Verfahren gefunden, nach welchem sich am Stickstoff substituierte 2-Ketosulfonamide in einfacher Weise dadurch herstellen lassen, daß man bestimmte 3-Amino-2-alkensäureester in Gegenwart einer Base mit Halogensulfonamiden umsetzt und das erhaltene Reaktionsprodukt verseift und decarboxyliert.

Gegenstand der Erfindung ist ein Verfahren zur Herstellung von 2-Keto-sulfonamiden der Formel I

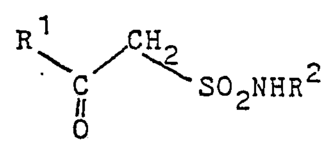

(I)

worin

$R^1$ einen $C_1$-$C_{11}$-Alkylrest, einen gegebenenfalls durch Halogenatome, $C_1$-$C_4$-Alkyloxy-, Nitro- oder $C_1$-$C_2$-Alkoxycarbonylgruppen substituierten Phenylrest, wobei die Anzahl der Substituenten am Phenylrest maximal drei ist, oder einen Phenyl-$C_1$-$C_2$-alkylrest, in dem die Phenylgruppe durch ein oder zwei Halogenatome oder $C_1$-$C_2$-Alkoxygruppen substituiert sein kann, und

$R^2$ ein Wasserstoffatom, einen $C_1$-$C_{12}$-Alkylrest oder einen $C_6$-$C_{12}$-Arylrest, welcher durch null bis 3 Hetero-atome und null bis 2 Kohlenstoffatome enthaltende Gruppen substituiert sein kann, bedeuten, dadurch gekennzeichnet, daß man einen 3-Amino-2-alkensäureester der Formel II

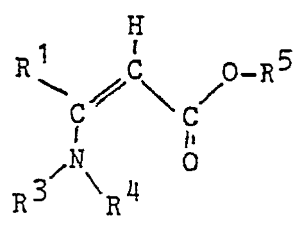

(II)

worin $R^1$ die vorgenannte Bedeutung hat und $R^3$ und $R^4$ gleich oder verschieden sind und Wasserstoff, $C_1$-$C_4$-Alkylreste, welche auch ringförmig miteinander verknüpft sein können, oder $C_7$-$C_{12}$-Aralkyl- oder $C_6$-$C_{12}$-Arylreste mit null bis 2 Heteroatomen und $R^5$ einen verzweigten oder unverzweigten $C_1$-$C_{18}$-Alkylrest, vorzugsweise $C_1$-$C_4$-Alkylrest, welcher Heteroatome enthalten kann, bedeuten mit einem Amidosulfohalogenid der Formel III

$$X{-}SO_2NH{-}R^2 \qquad (III),$$

worin $R^2$ die obengenannte Bedeutung hat und X Chlor, Brom oder Fluor bedeutet, in Gegenwart mindestens eines Äquivalents, bezogen auf den eingesetzten Ester, einer Base umsetzt und den erhaltenen 3-Amino-2-sulfamoyl-2-alkensäureester der Formel IV

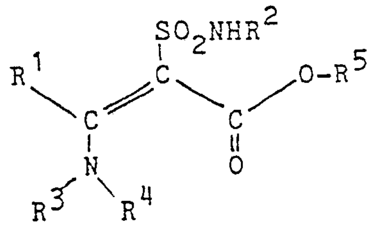

(IV)

worin $R^1$, $R^2$, $R^3$, $R^4$ und $R^5$ die obengenannte Bedeutung haben, einer sauren oder alkalischen Verseifung und Decarboxylierung unterwirft.

Ein weiterer Gegenstand der Erfindung sind 2-Keto-sulfonamide der Formel I

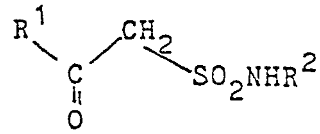

(I)

worin

$R^1$ einen $C_1$-$C_{11}$-Alkylrest, einen gegebenenfalls durch Halogenatome, $C_1$-$C_4$-Alkyloxy-, oder $C_1$-$C_2$-Alkoxycarbonylgruppen substituierten Phenylrest, wobei die Anzahl der Substituenten am Phenylrest maximal drei ist, oder einen Phenyl-$C_1$-$C_2$-alkylrest, in dem die Phenylgruppe durch ein oder zwei Halogenatome oder $C_1$-$C_2$-Alkoxygruppen substituiert sein kann, und

$R^2$ ein Wasserstoffatom, einen $C_1$-$C_{12}$-Alkylrest oder einen $C_6$-$C_{12}$-Arylrest, welcher durch null bis 3 Heteroatome und null bis 2 Kohlenstoffatome enthaltende Gruppen substituiert sein kann, bedeuten, ausgenommen die Verbindungen der Formel I, in welcher

$R^1$ ein $C_1$-$C_4$-Alkyl- oder Phenylrest ist und $R^2$ Wasserstoff bedeutet, und

$R^1$ ein Phenylrest ist und $R^2$ einen Phenyl-, Cyclohexyl- oder n-Butylrest bedeutet.

Ein weiterer Gegenstand der Erfindung ist ein Verfahren zur Herstellung von 3-Amino-2-sulfamoyl-2-alkensäureestern der der Formel IV

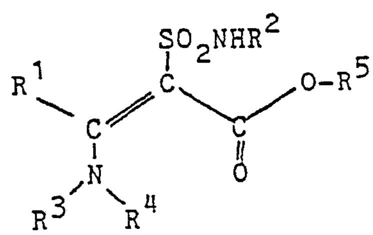

(IV)

$R^1$ einen $C_1$-$C_{11}$-Alkylrest, einen gegebenenfalls durch Halogenatome, $C_1$-$C_4$-Alkyloxy-, Nitro- oder $C_1$-$C_2$-Alkoxycarbonylgruppen substituierten Phenylrest, wobei die Anzahl der Substituenten am Phenylrest maximal drei ist, oder einen Phenyl-$C_1$-$C_2$-alkylrest, in dem die Phenylgruppe durch ein oder zwei Halogenatome oder $C_1$-$C_2$-Alkoxygruppen substituiert sein kann, und

$R^2$ ein Wasserstoffatom, einen $C_1$-$C_{12}$-Alkylrest oder einen $C_6$-$C_{12}$-Arylrest, welcher durch null bis 3 Heteroatome und null bis 2 Kohlenstoffatome enthaltende Gruppen substituiert sein kann, bedeuten,

$R^3$ und $R^4$ gleich oder verschieden sind und Wasserstoff, $C_1$-$C_4$-Alkylreste, welche auch ringförmig miteinander verknüpft sein können, oder $C_7$-$C_{12}$-Aralkyl- oder $C_6$-$C_{12}$-Arylreste mit null bis zwei Heteroatomen und $R^5$ einen verzweigten oder unverzweigten $C_1$-$C_{18}$-Alkylrest, welcher Heteroatome enthalten kann, bedeuten,

dadurch gekennzeichnet, daß man einen 3-Amino-2-alkensäureester der Formel II

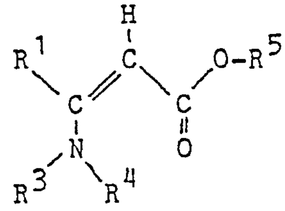

(II)

worin $R^1$, $R^3$, $R^4$ und $R^5$ die vorgenannte Bedeutung haben mit einem Amidosulfohalogenid der Formel III

$$X{-}SO_2NH{-}R^2 \qquad (III),$$

worin $R^2$ die obengenannte Bedeutung hat und X Chlor, Brom oder Fluor bedeutet, in Gegenwart

mindestens eines Äquivalents, bezogen auf den eingesetzten Ester, einer Base umsetzt.

Ausgangsstoffe für das erfindungsgemäße Verfahren sind 3-Amino-2-alkensäureester der Formel II

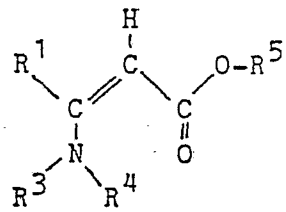

(II)

worin $R^1$ die oben angegebene Bedeutung hat, $R^3$ und $R^4$ gleich oder verschieden sind und Wasserstoff, $C_1$-$C_4$-Alkylreste, welche auch ringförmig miteinander verknüpft sein können, oder $C_7$-$C_{12}$-Aralkyl- oder $C_6$-$C_{12}$-Arylreste mit null bis 2 Heteroatomen und $R^5$ einen verzweigten oder unverzweigten $C_1$-$C_{18}$-Alkylrest, vorzugsweise $C_1$-$C_4$-Alkylrest, welcher Heteroatome enthalten kann, beispielsweise 2-Methoxyethyl- oder 2-Chlorethyl-, bedeuten. Diese Verbindungen können hinsichtlich der C = C-Doppelbindung in E- und Z-Konfiguration vorliegen.

Geeignet sind beispielsweise 3-Methylamino-, Dimethylamino-, Ethylamino-, Diethylamino-, Hydroxyethylamino-, i-Propylamino-, Butylamino-, Dibutylamino-, Pyrrolidino-, Piperidino-, Benzylamino-, Phenylamino-, Diphenylamino-, Biphenylamino-, 2,3- oder 4-Chlorphenyl-amino-, 2,3- oder 4-Methoxyphenylamino-2-alken-säureester.

Besonders bevorzugt werden für das erfindungsgemäße Verfahren 3-Amino-crotonsäureester als Ausgangsstoffe verwendet.

Die besten Ausbeuten werden mit 3-Aminocrotonsäureestern erhalten, die am Stickstoffatom noch mindestens 1 Wasserstoffatom tragen.

Um gut kristallisierende Reaktionsprodukte zu erhalten, ist es vorteilhaft, solche 3-Aminocrotonsäureester als Ausgangsmaterial zu verwenden, die selbst bereits gut kristallisieren. Besonders geeignet sind beispielsweise 3-Amino- und 3-Anilinocrotonsäureester.

Die 3-Amino-2-alkensäureester der Formel (II) werden mit einem Amidosulfohalogenid der Formel (III).

$$X—SO_2NH—R^2 \qquad (III),$$

worin $R^2$ die obengenannte Bedeutung hat und X Chlor, Brom oder Fluor, vorzugsweise Chlor, bedeutet, umgesetzt.

Je nach Wahl des Substituenten $R^2$ in dem Amidosulfohalogenid ist es nach dem erfindungsgemäßen Verfahren möglich, eine große Zahl am Stickstoff unterschiedlich substituierter 2-Ketosulfonamide herzustellen. Geeignete Ausgangsmaterialien sind beispielsweise Methylamido-, Ethylamido-, 1-Propylamido-, tert.-Butylamido-, n-Butylamido-, Hexylamido-, Phenylamido-, 2-Chlorphenylamido-, 3-Methoxyphenylamido-, 4-Methylphenylamido-, Diphenylamido-, 2,4-Diethoxyphenylamido-, 3,4,5-Triethoxyphenylamido-, 4-Nitrophenylamidosulfochlorid.

Besonders bevorzugt ist das am Stickstoff unsubstituierte Amidosulfochlorid, das aus Chlorsulfonylisocyanat besonders leicht zugänglich ist (R. Graf, Angew. Chem. *80*, 180 (1968)).

Bei der Umsetzung der 3-Amino-2-alkensäureester mit dem Amidosulfohalogenid ist der Zusatz von mindestens einem Äquivalent einer Base erforderlich, die den freiwerdenden Halogenwasserstoff bindet. Hierzu kann ein Überschuß von 3-Amino-2-alkensäureester verwendet werden. Vorteilhafter ist der Einsatz einer billigen oder leicht regenerierbaren Hilfsbase. Geeignet sind insbesondere alle tertiären oder sterisch gehinderten Amine, wie beispielsweise Trimethylamin, Triethylamin, Triethylendiamin (DABCO), Pyridin, Dimethylamin, Di-cyclohexylamin, Triphenylamin, N-Methylpiperidin.

Die Reaktion kann aber auch in Gegenwart von anorganischen Basen der Alkali- oder Erdalkalimetalle wie Lithiumhydroxid, Natriumhydroxid, Natriumcarbonat, Natriumhydrogencarbonat, Kaliumhydroxid, Kaliumcarbonat, Kaliumhydrogencarbonat, Calciumhydroxid, Magnesiumhydroxid, in Festsubstanz, in Lösung oder unter Phasentransferbedingungen durchgeführt werden.

Die Zugabe der Reaktanden erfolgt in beliebiger Reihenfolge gleichzeitig oder nacheinander. Vorteilhaft ist es, das besonders reaktionsfähige Amidosulfochlorid vorzulegen und die Base und den 3-Amino-2-alkensäureester gleichzeitig getrennt oder in Mischung zulaufen zu lassen. Vorteilhaft verwendet man etwa äquimolare Mengen der Komponenten. Überschüsse von Reaktanden bringen keinen Vorteil. Man vereinigt die Komponenten vorteilhaft im Temperaturbereich zwischen — 10 und + 40 °C. Unterhalb dieses Bereiches verläuft die Reaktion sehr langsam, oberhalb derselben nimmt die Ausbeute allmählich ab.

Man kann in An- oder Abwesenheit indifferenter Lösungs- oder Verdünnungsmitteln einzeln oder in Mischungen arbeiten. Als Lösungsmittel geeignet sind beispielsweise : Kohlenwasserstoffe wie Pentan, Hexan, Leichtbenzin, Ligroin, Benzol, Toluol, Xylol ; Chlorkohlenwasserstoffe wie Methylenchlorid, Tetrachlorethylen, Tetrachlorkohlenstoff, Chloroform, Chlorpentan, Chlorbenzol, Dichlorbenzol ; Ether

wie Dimethylether, Diethylether, Di-i-propylether, Phenylethylether, Dioxan, Tetrahydrofuran, Dimethoxyethan. Carbonsäurederivate wie Ethylacetat, Acetonitril, Dimethylformamid.

Weitere geeignete Lösungsmittel sind beispielsweise Phosphorsäuretrisdimethylamid oder flüssiges Schwefeldioxid, gegebenenfalls unter Druck.

Die erforderliche Reaktionszeit hängt von der Reaktionsfreudigkeit der Komponenten und der Reaktionstemperatur ab. Sie muß von Fall zu Fall ermittelt werden. Durch Wahl der Reaktionsbedingungen wird sie vorteilhaft auf 2-24 Stunden beschränkt.

Bei der Reaktion erhält man 3-Amino-2-sulfamoyl-2-alkensäureester der Formel (IV)

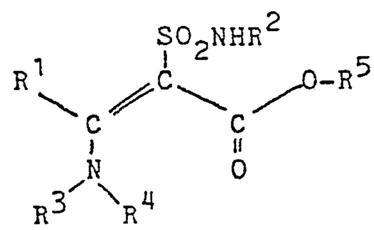

(IV)

worin $R^1$, $R^2$, $R^3$, $R^4$ und $R^5$ die obengenannte Bedeutung haben.

Bevorzugt werden diejenigen 3-Amino-2-sulfoamoyl-2-alkensäureester der Formel (IV), bei denen $R^1$ einen $C_1$-$C_4$-Alkylkrest, einen Phenyl- oder einen durch ein oder zwei Chloratome substituierten Phenylrest oder einen Benzylrest, $R^2$ ein Wasserstoffatom, einen geradkettigen $C_1$-$C_4$-Alkylrest oder einen Phenylrest, $R^3$ ein Wasserstoffatom, eine Methylgruppe oder einen Phenylrest, $R^4$ ein Wasserstoffatom und $R^5$ einen $C_1$-$C_4$-Alkylrest bedeuten.

Die Zwischenprodukte der Formel (IV) werden sauer oder alkalisch verseift und decarboxyliert. Dabei werden die literaturbekannten Bedingungen zur Verseifung von Enaminen und Estern und zur Decarboxylierung von Acetessigsäurederivaten angewandt. Man kann beispielsweise zunächst in alkalischem Medium die Estergruppe verseifen und anschließend sauer decarboxylieren und die Enamingruppe verseifen. Ebenso kann man in umgekehrter Reihenfolge verfahren. Man kann weiterhin Verseifung von Ester- und Enamingruppe sowie Decarboxylierung in einer Stufe sauer durchführen.

Man kann ebenso die Estergruppe in Dimethylsulfoxids in Gegenwart von Alkalihalogeniden bei erhöhter Temperatur abspalten.

Zur Verseifung sind mindestens die stöchiometrischen Mengen Wasser erforderlich. Säuren und Basen können in konzentrierter oder verdünnter Lösung oder in Substanz zugesetzt werden. Vorteilhaft arbeitet man in wäßrigen Medien. Dem Reaktionsgemisch können Lösungs- oder Verdünnungsmittel wie Methanol, Ethanol, Aceton, Methylenchlorid, Benzol, Toluol, Chlorbenzol zugesetzt werden.

Verseifung und Decarboxylierung können in weitem Temperaturbereich durchgeführt werden. Die Abspaltung der Estergruppe in DMSO wird in der Regel Bei 100-180 °C durchgeführt, die Enamin- und Esterverseifung verläuft bereits bei Temperaturen um 0 °C verhältnismäßig rasch. Bei 80-120 °C ist die Spaltung und Verseifung des tert.-Butylesters in 1-2 Stunden beendet.

Bei der sauren Hydrolyse der 3-Amino-2-sulfamoyl-2-alkensäureestern können als Zwischenprodukte 2-Sulfamoyl-3-ketoalkansäureester der Formel (V) isoliert werden

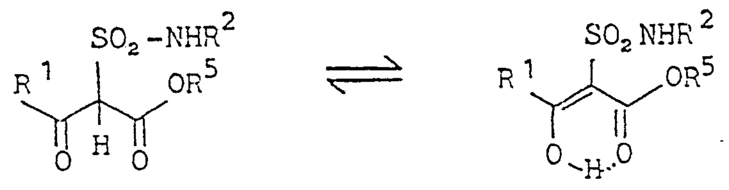

(V)

worin $R^1$, $R^2$ und $R^5$ die oben angegebene Bedeutung haben.

Diese Verbindungen können, wie im Formelbild angegeben, in zwei tautomeren Formen vorliegen, wie von β-Ketocarbonsäureestern bekannt ist.

Unter den 2-Sulfamoyl-3-keto-alkansäureestern der Formel (V) sind solche bevorzugt, bei denen $R_1$ eine $C_1$-$C_4$-alkylgruppe, einen Phenyl- oder einen durch ein oder zwei Chloratome substituierten Phenylrest oder einen Benzylrest, $R^2$ ein Wasserstoffatom und $R^5$ einen $C_1$-$C_4$-Alkylrest bedeuten.

Die bevorzugten Verbindungen der Formel (IV) und (V) zeichnen sich u. a. durch besonders gute Zugänglichkeit aufgrund der besseren Ausbeuten und Kristallisationstendenz aus.

Besonders vorteilhaft ist die Verwendung der 3-Aminocrotonsäure-tert.-butylester. Sie lassen sich besonders leicht in einer Stufe zum 2-Ketosulfonamid verseifen und unter Abspaltung von $CO_2$ und i-Buten decarboxylieren.

Die erfindungsgemäßen 2-Ketosulfonamide der Formel (I) sind Vorprodukte für neue Pflanzenschutzmittel und Pharmaka. Sie stellen jedoch Analoga der Acylessigsäureamide dar, die ihrerseits große Bedeutung als Kupplungskomponenten für Azofarbstoffe und -pigmente bestitzen.

Die folgenden Beispiele sollen die Erfindung erläutern.

Beispiel 1

2-Amidosulfonyl-3-aminocrotonsäure-tert. butylester

In die Lösung von

578 g (5 Mol) Amidosulfochlorid in
2 500 ml Acetonitril (über Molekularsieb getrocknet) tropfte man bei 0 bis 5 °C eine Mischung von
785 g (5 Mol) 3-Aminocrotonsäure-tert.-butylester
510 g (5,05 Mol) Triethylamin und
500 ml Acetonitril.

Nach 2-stündigem Rühren bei gleicher Temperatur und weiteren 6 Stunden bei Raumtemperatur setzte man unter Eiskühlung 835 ml 6n Natronlauge zu, rührte eine Stunde, trennte ab und extrahierte die wäßrige Schicht noch 2x mit Acetonitril. Nach Eindampfen der Acetonitril-Lösung im Rotationsverdampfer erhielt man einen Rückstand, der aus Methanol/Wasser umkristalliesiert wurde.
Ausbeute : 1 004 g = 85 % (d. Th.) ;
Fp 134-135 °C (Zers.)

Das gleiche Ergebnis erhielt man, als man anstelle von Triethylamin die äquivalente Menge 3-Aminocrotonsäure-tert.-butylester, Dimethylanilin oder Pyridin verwendete.

Acetonsulfonamid

236 g (1 Mol) 2-Amidosulfonyl-3-amino-crotonsäure-tert.-butylester wurden in 1 000 ml 2 n Salzsäure unter Rückfluß gekocht, bis alles gelöst war und die Gasentwicklung aufhörte (ca. 2 Stunden).
Nach dem Eindampfen wurde der Rückstand mit Acetonitril extrahiert und die erhaltene Lösung erneut im Rotationsverdampfer eingedampft. Der kristallisierende Rückstand (140 g) wurde aus i-Propanol umkristallisiert. Man erhielt 122 g (89 % d. Th.) Acetonsulfoamid, Fp 74-76 °C (i-Propanol).

Beispiel 2

2 Amidosulfonyl-3-amino-crotonsäureethylester

In 50 ml einer 2 molaren Lösung von Amidosulfochlorid in wasserfreiem Acetonitril wurde bei — 2 °C-0 °C eine Lösung von 26 g (0.2 Mol) 3-Aminocrotonsäureethylester in 70 ml abs. Acetonitril rasch eingetropft. Man rührte 45 Minuten unter Eiskühlung, 30 Minuten bis zum Erreichen der Raumtemperatur und 1 Stunde bei Raumtemperatur nach, kühlte auf ca. 1 °C ab und tropfte bei 1 °C-3 °C 16.5 ml 6n NaOH zu. Die resultierende Mischung wude im Vakuum bei 35 °C Badtemperatur auf ein Rückstandsgewicht von 50 bis 55 g eingeengt, mit 30 ml Wasser versetzt und mehrmals mit Ethylacetat extrahiert. Die vereinigten Extrakte wurden mit $Na_2SO_4$ getrocknet, filtriert und im Vakuum eingedampft. Der verbleibende Rückstand (32-35 g) wurde mit ca. 100 ml Diisopropylether durchmischt. Der Diisopropylether-Extrakt wurde von der zähöligen, teils kristallinen Masse abgegossen. Diese wurde in 120 ml siedendem Ethylacetat gelöst. Die heiße Lösung filtrierte man über Filterhilfe. Im Filtrat kristallisierte beim Kühlen (Eisbad) das Produkt aus. Nach dem Absaugen und Auswaschen (mit AcOEt) des kristallinen Anteils konnte durch Einengen der Mutterlauge eine weitere Menge an reinem Produkt erhalten werden. Man erhielt so 15.6 g (75 %) 2-Amidosulfonyl-3-amino-crotonsäureethylester.
Fp 103-104 °C.
Anstelle von Acetonitril konnte auch 1.2-Dimethoxyethan (Glyme) als Lösungsmittel verwendet werden.
Nach der gleichen Arbeitsweise wie oben beschrieben wurden erhalten : 2-Amidosulfonyl-3-methylamino-crotonsäureethylester, Fp 69-70 °C (aus Ethylacetat/Diisopropylether) ; Ausbeute : ca. 70 % ; α-Amidosulfonyl-β-amino-zimtsäuremethylester, Fp 141-142 °C (aus $CH_2Cl_2$), Ausbeute : ca. 70 % ; in diesem Fall wurde als organisches Extraktionsmittel eine größere Menge $CH_2Cl_2$, aus dem beim Einengen die Amidosulfonyl-Verbindung sogleich rein anfiel, verwendet. Es konnte auch Ethylacetat als Extraktionsmittel verwendet werden.

Acetonsulfonamid

20,8 g 2-Amidosulfonyl-3-amino-crotonsäureethylester wurden mit 200 ml 2 n Natronlauge 18 Stunden bei Raumtemperatur gerührt. Man stellte mit konz. Salzsäure auf pH 1 ein, setzte nochmals 0,2 Mol konz. Salzsäure zu und kochte unter Rückfluß bis zur beendeten Gasentwicklung. Nach Eindampfen

der Lösung wurde der trockene Rückstand mit Acetonitril extrahiert. Aus dem Extrakt erhielt man nach erneutem Eindampfen 5,4 g (39 % d. Th.) Aceton-N-sulfonamid.

Fp 74-76° (i-Propanol).

Beispiel 3

2-N-Methylamidosulfonyl-3-aminocrotonsäure-tert.-butylester

In die Lösung von

129,5 g (1 Mol) N-Methylamidosulfochlorid in
500 ml Methylenchlorid tropfte man bei 0 bis 5 °C
157 g (1 Mol) 3-Aminocrotonsäure-tert.-butylester und
105 g (1,04 Mol) Triethylamin in
100 ml Methylenchlorid. Nach Stehenlassen des Ansatzes über Nacht bei Raumtemperatur schüttelte man ihn 3 mal mit Wasser aus, dampfte die organische Lösung ein, verrieb den kristallisierenden Rückstand mit i-Propylether und saugte ab.

Ausbeute : 200 g (80 % d. Th.)

Fp 102 °C (Methanol/Wasser).

Aceton-N-methylsulfonamid

Das Reaktionsprodukt wurde mit 800 ml 2n Salzsäure unter Rückfluß gekocht, bis alles gelöst war und die Gasentwicklung aufhörte (ca. 2 Stunden). Nach dem Eindampfen im Rotationsverdampfer wurde der Rückstand mit Acetonitril extrahiert und der Extrakt erneut eingedampft. Man erhielt 115 g (95 % d. Th.) Aceton-N-methylsulfonamid.

Fp 42 °C (Methanol/$H_2O$)

Beispiel 4

2-N-Ethylamidosulfonyl-3-anilino-crotonsäure-tert.-butylester

Zu

143,5 g (1 Mol) N-Ethylamidosulfochlorid, gelöst in
500 ml Methylenchlorid, tropfte man bei 0-5 °C eine Lösung von
233,6 g (1 Mol) 3-Anilinocrotonsäure-tert.-butylester und
101 g (1 Mol) Triethylamin in 200 ml Methylenchlorid. Man rührte 1 Stunde bei gleicher Temperatur und weitere 6 Stunden bei Raumtemperatur nach, schüttelte 2 mal mit Wasser aus und dampfte die Methylenchloridlösung ein. Man erhielt 310 g (91 % d. Th.) 2-N-Ethylamidosulfonyl-3-anilinocrotonsäure-tert.-butylester.

Fp 65 °C (Methanol)

Aceton-N-ethylsulfonamid

Das Reaktionsprodukt wurde mit 500 ml 2 n-Salzsäure ca. 2 Stunden unter Rückfluß gekocht. Nach beendeter Gasentwicklung wurde mit 33 %iger Natronlauge auf pH 11 gestellt und mit i-Propylether extrahiert. Die wäßrige Lösung wurde mit Salzsäure erneut angesäuert und im Vakuum eingedampft. Durch Extrahieren mit Acetonitril und Eindampfen des Extraktes erhielt man 138 g (92 % d. Th.) Aceton-N-ethylsulfonamid.

Fp 57 °C (i-Propanol).

Beispiel 5

2-N-Phenylamidosulfonyl-3-anilino-crotonsäure-tert.-butylester

Zu

38 g (0,2 Mol) Phenylamidosulfochlorid, gelöst in
200 ml Methylenchlorid tropfte man bei 0-5 °C eine Lösung von
46,6 g (0,2 Mol) 3-Anilinocrotonsäure-tert.-butylester und
21,0 g (0,21 Mol) Triethylamin in
100 ml Methylenchlorid. Man hielt 1 Stunde bei 5 °C und ließ über Nacht bei Raumtemperatur stehen. Die Methylenchloridlösung wurde nach Ausschütteln mit Wasser eingedampft. Man erhielt 72,4 g (93 % d. Th) kristallisiertem Rückstand.

Fp 108 °C ; (Methanol).

Aceton-N-phenylsulfonamid

19,4 g (50 mMol) Reaktionsprodukt wurden mit 50 ml 2-n-Salzsäure und 30 ml Ethanol bis zur beendeten Gasentwicklung (1 Stunde) unter Rückfluß gekocht. Nach Abkühlen kristallisierten 5 g Aceton-N-phenylsulfonamid aus. Durch Einengen der Mutterlauge erhielt man weitere 4 g. Gesamtausbeute 84 % d. Th.

Fp 92 °C (Methanol).

Beispiel 6

Zu einer Lösung von

16 g (0,1 Mol) Phenylamidosulfochlorid in
50 ml Methylenchlorid tropfte man bei 30 °C
22,7 g (0,1 Mol) 3-Morpholino-crotonsäure-tert.-butylester und
11 g (0,11 Mol) Triethylamin, gelöst in
20 ml Methylenchlorid. Nach 6 Stunden wurde mit Wasser ausgeschüttelt, Methylenchlorid abdestilliert und der Rückstand mit
100 ml 2n Salzsäure bis zur beendeten Gasentwicklung gekocht. Aus dem Reaktionsgemisch wurde Aceton-N-phenylsulfonamid chromatographisch mit einem Gemisch von 75 Vol.% Hexan und 25 Vol.% Ethylacetat auf Silicagel abgetrennt.

Beispiel 7

2-Amidosulfonyl-3-amino-hex-2-ensäureethylester

Zu einer Lösung von 4,10 g (35,5 mMol) Amidosulfochlorid in 20 ml wasserfreiem Acetonitril wurde in ca. 15 Minuten bei —5 °C eine Lösung von 10,72 g (68,2 mMol) 3-Aminohex-2-ensäureethylester in 20 ml wasserfreiem Acetonitril zugetropft. Man rührte 45 Minuten bei 0°-2 °C, 45 Minuten bei 1 °C bis Raumtemperatur und 1,5 Stunden bei Raumtemperatur, tropfte bei ca. 4 °C 6,4 ml 6n NaOH zu und dampfte die Reaktionsmischung bei 38 °C Badtemperatur auf ein Rückstandsgewicht von ca. 20 g ein. Diesen Rückstand nahm man in einer Mischung von 7 ml Wasser und ca. 100 ml $CH_2Cl_2$ auf, trennte nach dem Durchmischen die Phasen und schüttelte die wäßrige noch mehrmals mit $CH_2Cl_2$ aus. Die vereinigten Extrakte wurden mit $Na_2SO_4$ getrocknet, filtriert und in Vakuum eingedampft. Den verbleibenden Rückstand löste man in Ethylacetat. Beim Zusatz von Diisopropylether erfolgte Kristallisation. Die kristalline Masse wurde abgesaugt, mit Diisopropylether gewaschen und im Vakuum getrocknet. Man erhielt so 7,60 g reinen 2-Amidosulfonyl-3-amino-hex-2-ensäureethylester, Fp 102-103 °C (DC rein), = 90,6 % Ausbeute.

2-Oxo-pentan-1-sulfonamid

2,36 g (10 mMol) 2-Amidosulfonyl-3-amino-hex-2-ensäureethylester wurden bei Raumtemperatur in 15 ml 2n Natronlauge gelöst. Nach 16 Stunden extrahierte man ölige Nebenprodukte mit Methylenchlorid, säuerte die wäßrige Lösung mit überschüssiger konz. Salzsäure an und erwärmte 15 Minuten auf 90 °C. Nach beendeter Gasentwicklung dampfte man ein und extrahierte den trockenen Rückstand mit siedendem Ethylacetat. Nach Eindampfen der Ethylacetatlösung erhielt man 0,76 g (58 % d. Th.) kristallisiertes 2-Oxo-pentan-1-sulfonamid vom Fp 117 °C.

Beispiel 8

2-Amidosulfonyl-3-keto-hexansäureethylester

Eine Mischung aus 3,54 g (15 mMol) 2-Amidosulfonyl-3-aminohex-2-ensäureethylester und 42 ml 4n HCl wurde 4 Stunden bei 32-34 °C gerührt. Nach Kühlung auf unter 7 °C wurde das Kristallisat abgesaugt und mit Wasser (benötigt wurden etwa 30 ml) neutral gewaschen. Das wäßrigsaure Filtrat wurde im Vakuum auf ein Volumen von ca. 20 ml eingeengt, wobei weiteres Kristallisat ausfiel, das abgesaugt und mit wenig Wasser gewaschen wurde. Man erhielt nach dem Trocknen der beiden kristallinen Anteile 3,36 g (= 94,4 % d. Th.) reinen 2-Amidosulfonyl-3-ketohexansäureethylester, Fp 77-78 °C.

Beispiel 9

2-Amidosulfonyl-acetessigsäureethylester

Eine Mischung aus 10,4 g 2-Amidosulfonyl-3-amino-crotonsäureethylester und 65 ml 4n HCl wurde 1 Stunde bei 30 °C und 3 Stunden bei Raumtemperatur gehalten und anschließend auf 36 g Rückstandsgewicht in Vakuum eingeengt. Nach 17 Stunden Stehen bei ~ 4 °C wurde die auskristallisierte Substanz abgesaugt und mit Wasser nachgewaschen. Hierbei fielen 6,6 g reiner 2-Amidosulfonyl-acetessigsäureethylester an. Die wäßrige Mutterlauge wurde im Vakuum bis auf 15 g Rückstand eingeengt und dann mit $CH_2Cl_2$ extrahiert. Die vereinigten $CH_2Cl_2$-Extrakte hinterließen nach dem Trocknen, Filtrieren und Eindampfen 3,5 g kristallinen Rückstand, der aus Ethylacetat/Diisopropylether (3 : 5) umkristallisiert 2,8 g reinen 2-Amidosulfonyl-acetessigsäureethylester (Fp 65-66 °C) ergab. Die Ausbeute an 2-Amidosulfonylacetessigsäureethylester betrug somit 9,4 g (= 90 %).

## Beispiel 10

2-Amidosulfonyl-benzoylessigsäuremethylester

Eine Mischung aus 2,57 g α-Amidosulfonyl-β-amino-zimtsäuremethylester und 20 ml 4n HCl wurde 2,5 Stunden bei Raumtemperatur gerührt. Anschließend saugte man das Kristallisat ab und wusch es mit Wasser nach. Nach dem Trocknen erhielt man 2,40 g (= 93 %) reinen 2-Amidosulfonyl-benzoylessigsäuremethylester vom Fp 164-165 °C.

## Beispiel 11

2-Amidosulfonyl-3-amino-4-phenyl-crotonsäureethylester

Zu einer Lösung von 3,10 g (26,8 mMol) Amidosulfochlorid in 20 ml wasserfreiem Acetonitril wurde in ca. 15 Minuten bei — 5 °C eine Lösung von 10,8 g (50,1 mMol) 3-Amino-4-phenyl-crotonsäureethylester in 40 ml wasserfreiem Acetonitril zugetropft. Man rührte 45 Minuten bei 0 °C bis 2 °C, 45 Minuten bei 2 °C bis Raumtemperatur und 2 Stunden bei Raumtemperatur nach, tropfte bei ca. 2 °C 4,8 ml 6n NaOH zu und dampfte die Mischung bei 38 °C Badtemperatur auf ein Rückstandsgewicht von ca. 15 g ein. Diesen Rückstand nahm man in einer Mischung von 12 ml Wasser und ca. 100 ml $CH_2Cl_2$ auf, trennte nach dem Durchmischen die Phasen und schüttelte die wäßrige noch 4 × mit $CH_2Cl_2$ aus. Die vereinigten Extrakte wurden mit $Na_2SO_4$ getrocknet, filtriert und im Vakuum eingedampft. Der verbleibende Rückstand (12,4 g) wurde an einer Kieselgel-Säule (Kieselgel S ; Korngröße 0,006 3-0,2 mm ; Säulen-Ø 24 mm, H 520 mm ; Lieferant Riedel de Haen) chromatographiert. Als Elutionsmittel diente anfangs $CH_2Cl_2$/Petrolether 1 : 1, womit überschüssiger 3-Amino-4-phenyl-crotonsäure- bzw. 4-Phenylacetessigsäureethylester eluiert wurde. Der $CH_2Cl_2$-Anteil im Elutionsmittel wurde kontinuierlich erhöht, bis nach einigen Zwischenfraktionen (1 600 ml Eluat) mit $CH_2Cl_2$ der 2-Amidosulfonyl-3-amino-4-phenyl-crotonsäureethylester eluiert wurde. An reinem 2-Amidosulfonylester wurden 4,74 g (= 66,7 %) als zähes farbloses Öl (E/Z-Mischung) erhalten :

$C_{12}H_{16}N_2O_4S$ :

| | | | | |
|---|---|---|---|---|
| Ber. : | C 50,7 % ; | H 5,7 % ; | N 9,9 % ; | S 11,3 % |
| Gef. : | C | H | N | S |

DC ($CH_2Cl_2$/EtOH : 100/5) RF 0,40.

2-Oxo-3-phenyl-propan-1-sulfonamid

2,84 g (10 mMol) 2-Amidosulfonyl-3-amino-4-phenylcrotonsäureethylester wurden in 15 ml 2-n-Natronlauge bei Raumtemperatur gelöst. Nach 16 Stunden extrahierte man ölige Nebenprodukte mit Methylenchlorid, stellte die wäßrige Lösung mit konz. Salzsäure auf pH 1 und erwärmte 15 Minuten auf 90 °C. Nach Eindampfen der wäßrigen Lösung wurde der Rückstand mit Ethylacetat extrahiert und der über Natriumsulfat getrocknete Extrakt erneut eingedampft. Man erhielt 1,43 g (68 % d. Th.) kristallines 2-Oxo-3-phenyl-propan-1-sulfonamid.

## Beispiel 12

2-Amidosulfonyl-4-phenyl-acetessigsäureethylester

1,43 g (5 mMol) 2-Amidosulfonyl-3-amino-4-phenyl-crotonsäureethylester (gemäß Beispiel 11) wurden mit 45 ml 4n HCl versetzt und 2,5 Stunden bei 32-35 °C gerührt (die Mischung blieb dabei zweiphasig). Anschließend extrahierte man mit Ether. Der getrocknete Etherextrakt wurde filtriert und eingedampft. Dabei verblieben 1,24 g kristalliner Rückstand, bei dem es sich um bereits praktisch reinen 2-Amidosulfonyl-4-phenyl-acetessigsäureethylester handelte. Diese Substanz wurde mit wenig Hexan vermischt und abgesaugt. Nach dem Trocknen wurden 1,16 g (= 81,3 % Ausbeute) reiner 2-Amidosulfonyl-4-phenyl-acetessigsäureethylester von Fp 77-78 °C erhalten.

**Patentansprüche**

1. Verfahren zur Herstellung von 2-Keto-sulfonamiden der Formel I

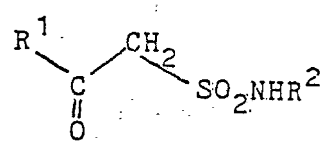

(I)

worin

$R^1$ einen $C_1$-$C_{11}$-Alkylrest, einen gegebenenfalls durch Halogenatome, $C_1$-$C_4$-Alkyloxy-, Nitro- oder $C_1$-$C_2$-Alkoxycarbonylgruppen substituierten Phenylrest, wobei die Anzahl der Substituenten am Phenylrest maximal drei ist, oder einen Phenyl-$C_1$-$C_2$-alkylrest, in dem die Phenylgruppe durch ein oder zwei Halogenatome oder $C_1$-$C_2$-Alkoxygruppen substituiert sein kann, und

$R^2$ ein Wasserstoffatom, einen $C_1$-$C_{12}$-Alkylrest oder einen $C_6$-$C_{12}$-Arylrest, welcher durch null bis 3 Heteroatome und null bis 2 Kohlenstoffatome enthaltende Gruppen substituiert sein kann, bedeuten, dadurch gekennzeichnet, daß man einen 3-Amino-2-alkensäureester der Formel II

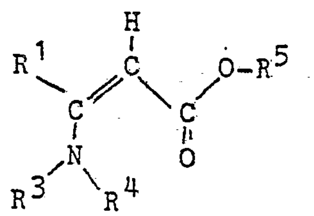

(II)

worin $R^1$ die vorgenannte Bedeutung hat und $R^3$ und $R^4$ gleich oder verschieden sind und Wasserstoff, $C_1$-$C_4$-Alkylreste, welche auch ringförmig miteinander verknüpft sein können, oder $C_7$-$C_{12}$-Aralkyl- oder $C_6$-$C_{12}$-Arylreste mit null bis 2 Heteroatomen und $R^5$ einen verzweigten oder unverzweigten $C_1$-$C_{18}$-Alkylrest, vorzugsweise $C_1$-$C_4$-Alkylrest, welcher Heteroatome enthalten kann, bedeuten mit einem Amidosulfohalogenid der Formel III

$$X—SO_2NH—R^2 \qquad (III)$$

worin $R^2$ die obengenannte Bedeutung hat und X Chlor, Brom oder Fluor bedeutet, in Gegenwart mindestens eines Äquivalents, bezogen auf den eingesetzten Ester, einer Base umsetzt und den erhaltenen 3-Amino-2-sulfamoyl-2-alkensäureester der Formel IV

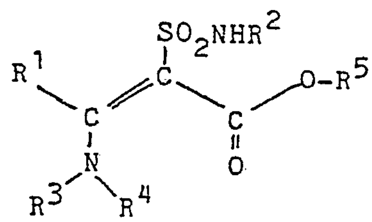

(IV)

worin $R^1$, $R^2$, $R^3$, $R^4$ und $R^5$ die obengenannte Bedeutung haben, einer sauren oder alkalischen Verseifung und Decarboxylierung unterwirft.

2. 2-Keto-sulfonamide der Formel I

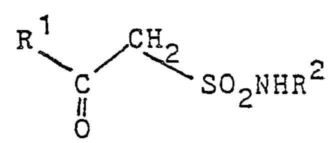

(I)

worin

$R^1$ einen $C_1$-$C_{11}$-Alkylrest, einen gegebenenfalls durch Halogenatome, $C_1$-$C_4$-Alkyloxy-, oder $C_1$-$C_2$-Alkoxycarbonylgruppen substituierten Phenylrest, wobei die Anzahl der Substituenten am Phenylrest maximal drei ist, oder einen Phenyl-$C_1$-$C_2$-alkylrest, in dem die Phenylgruppe durch ein oder zwei Halogenatome oder $C_1$-$C_2$-Alkoxygruppen substituiert sein kann, und

$R^2$ ein Wasserstoffatom, einen $C_1$-$C_{12}$-Alkylrest oder einen $C_6$-$C_{12}$-Arylrest, welcher durch null bis 3

Heteroatome und null bis 2 Kohlenstoffatome enthaltende Gruppen substituiert sein kann, bedeuten, ausgenommen die Verbindungen der Formel I, in welcher

$R^1$ ein $C_1$-$C_4$-Alkyl- oder Phenylrest ist und $R^2$ Wasserstoff bedeutet, und

$R^1$ ein Phenylrest ist und $R^2$ einen Phenyl-, Cyclohexyl- oder n-Butylrest bedeutet.

3. Verfahren zur Herstellung von 3-Amino-2-sulfamoyl-2-alkensäureestern der Formel IV

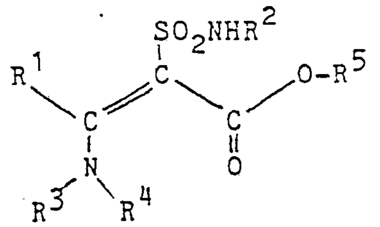

(IV)

$R^1$ einen $C_1$-$C_{11}$-Alkylrest, einen gegebenenfalls durch Halogenatome, $C_1$-$C_4$-Alkyloxy-, Nitro- oder $C_1$-$C_2$-Alkoxycarbonylgruppen substituierten Phenylrest, wobei die Anzahl der Substituenten am Phenylrest maximal drei ist, oder einen Phenyl-$C_1$-$C_2$-alkylrest, in dem die Phenylgruppe durch ein oder zwei Halogenatome oder $C_1$-$C_2$-Alkoxygruppen substituiert sein kann, und

$R^2$ ein Wasserstoffatom, einen $C_1$-$C_{12}$-Alkylrest oder einen $C_6$-$C_{12}$-Arylrest, welcher durch null bis 3 Heteroatome und null bis 2 Kohlenstoffatome enthaltende Gruppen substituiert sein kann, bedeuten,

$R^3$ und $R^4$ gleich oder verschieden sind und Wasserstoff, $C_1$-$C_4$-Alkylreste, welche auch ringförmig miteinander verknüpft sein können, oder $C_7$-$C_{12}$-Aralkyl- oder $C_6$-$C_{12}$-Arylreste mit null bis zwei Heteroatomen und $R^5$ einen verzweigten oder unverzweigten $C_1$-$C_{18}$-Alkylrest, welcher Heteroatome enthalten kann, bedeuten,

dadurch gekennzeichnet, daß man einen 3-Amino-2-alkensäureester der Formel II

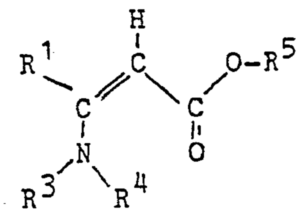

(II)

worin $R^1$, $R^3$, $R^4$ und $R^5$ die vorgenannte Bedeutung haben mit einem Amidosulfohalogenid der Formel III

$$X—SO_2NH—R^2 \qquad (III),$$

worin $R^2$ die obengenannte Bedeutung hat und X Chlor, Brom oder Fluor bedeutet, in Gegenwart mindestens eines Äquivalents, bezogen auf den eingesetzten Ester, einer Base umsetzt.

4. 3-Amino-2-sulfamoyl-2-alkensäureester der Formel IV

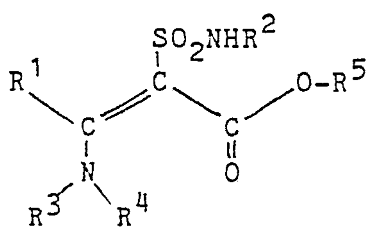

(IV)

$R^1$ einen $C_1$-$C_{11}$-Alkylrest, einen gegebenenfalls durch Halogenatome, $C_1$-$C_4$-Alkyloxy-, Nitro- oder $C_1$-$C_2$-Alkoxycarbonylgruppen substituierten Phenylrest, wobei die Anzahl der Substituenten am Phenylrest maximal drei ist, oder einen Phenyl-$C_1$-$C_2$-alkylrest, in dem die Phenylgruppe durch ein oder zwei Halogenatome oder $C_1$-$C_2$-Alkoxygruppen substituiert sein kann, und

$R^2$ ein Wasserstoffatom, einen $C_1$-$C_{12}$-Alkylrest oder einen $C_6$-$C_{12}$-Arylrest, welcher durch null bis 3 Heteroatome und null bis 2 Kohlenstoffatome enthaltende Gruppen substituiert sein kann, bedeuten,

$R^3$ und $R^4$ gleich oder verschieden sind und Wasserstoff, $C_1$-$C_4$-Alkylreste, welche auch ringförmig miteinander verknüpft sein können, oder $C_7$-$C_{12}$-Aralkyl- oder $C_6$-$C_{12}$-Arylreste mit null bis zwei Heteroatomen und $R^5$ einen verzweigten oder unverzweigten $C_1$-$C_{18}$-Alkylrest, welcher Heteroatome enthalten kann, bedeutet.

**Claims**

1. A process for the preparation of 2-ketosulfonamides of the formula I

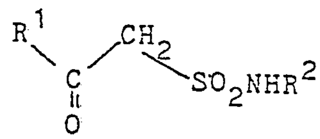

(I)

in which

$R^1$ denotes a $C_1$-$C_{11}$ alkyl radical, a phenyl radical optionally substituted by halogen atoms or $C_1$-$C_4$ alkoxy, nitro or $C_1$-$C_2$ alkoxycarbonyl groups, the number of substituents on the phenyl radical being at most three, or a phenyl-$C_1$-$C_2$-alkyl radical in which the phenyl group can be substituted by one or two halogen atoms or $C_1$-$C_2$ alkoxy groups, and

$R^2$ denotes a hydrogen atom, a $C_1$-$C_{12}$ alkyl radical or a $C_6$-$C_{12}$ aryl radical which can be substituted by groups containing 0 to 3 heteroatoms and 0 to 2 carbon atoms,
wherein a 3-aminoalk-2-enoic acid ester of the formula II

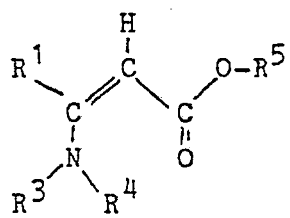

(II)

in which $R^1$ has the meaning given above, $R^3$ and $R^4$ are identical or different and denote hydrogen, $C_1$-$C_4$ alkyl radicals which can also be linked together to form a ring, or $C_7$-$C_{12}$ aralkyl or $C_6$-$C_{12}$ aryl radicals having 0 to 2 heteroatoms, and $R^5$ denotes a branched or unbranched $C_1$-$C_{18}$ alkyl radical, preferably a $C_1$-$C_4$ alkyl radical, which can contain heteroatoms, is reacted with an amidosulfonyl halide of the formula III

$$X—SO_2NH—R^2 \quad (III),$$

in which $R^2$ has the abovementioned meaning and X denotes chlorine, bromine or fluorine, in the presence of at least one equivalent, based on the ester used, of a base, and the resulting 3-amino-2-sulfamoylalk-2-enoic acid ester of the formula IV

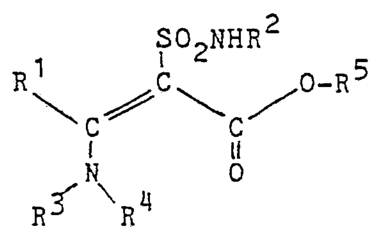

(IV)

in which $R^1$, $R^2$, $R^3$, $R^4$ and $R^5$ have the abovementioned meanings, is subjected to acid or alkaline saponification and decarboxylation.

2. A 2-ketosulfonamide of the formula I

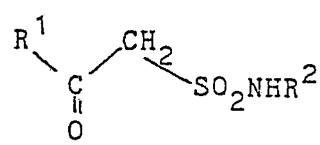

(I)

in which

$R^1$ denotes a $C_1$-$C_{11}$ alkyl radical, a phenyl radical optionally substituted by halogen atoms or $C_1$-$C_4$ alkoxy, or $C_1$-$C_2$ alkoxycarbonyl groups, the number of substituents on the phenyl radical being at most three, or a phenyl-$C_1$-$C_2$-alkyl radical in which the phenyl group can be substituted by one or two halogen atoms or $C_1$-$C_2$ alkoxy groups, and

$R^2$ denotes a hydrogen atom, a $C_1$-$C_{12}$ alkyl radical or a $C_6$-$C_{12}$ aryl radical which can be substituted by groups containing 0 to 3 heteroatoms and 0 to 2 carbon atoms,

with the exception of the compounds of the formula I in which

$R^1$ is a $C_1$-$C_4$ alkyl or phenyl radical and $R^2$ denotes hydrogen, and

$R^1$ is a phenyl radical and $R^2$ denotes a phenyl, cyclohexyl or n-butyl radical.

3. A process for the preparation of 3-amino-2-sulfamoylalk-2-enoic acid esters of the formula IV

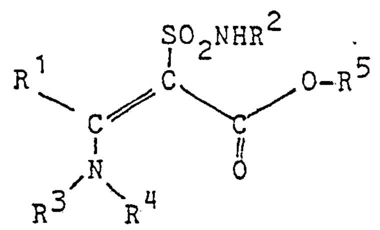

(IV)

in which

$R^1$ denotes a $C_1$-$C_{11}$ alkyl radical, a phenyl radical optionally substituted by halogen atoms or $C_1$-$C_4$ alkoxy, nitro or $C_1$-$C_2$ alkoxycarbonyl groups, the number of substituents on the phenyl radical being at most three, or a phenyl-$C_1$-$C_2$-alkyl radical in which the phenyl group can be substituted by one or two halogen atoms or $C_1$-$C_2$ alkoxy groups, and

$R^2$ denotes a hydrogen atom, a $C_1$-$C_{12}$ alkyl radical or a $C_6$-$C_{12}$ aryl radical which can be substituted by groups containing 0 to 3 heteroatoms and 0 to 2 carbon atoms,

$R^3$ and $R^4$ are identical or different and denote hydrogen, $C_1$-$C_4$ alkyl radicals which can also be linked together to form a ring, or $C_7$-$C_{12}$ aralkyl or $C_6$-$C_{12}$ aryl radicals having 0 to 2 heteroatoms, and $R^5$ denotes a branched or unbranched $C_1$-$C_{18}$ alkyl radical which can contain heteroatoms,

wherein a 3-aminoalk-2-enoic acid ester of the formula II

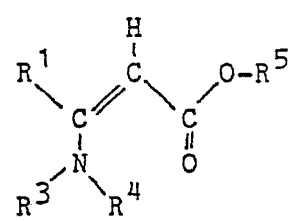

(II)

in which $R^1$, $R^3$, $R^4$ and $R^5$ have the abovementioned meanings, is reacted with an amidosulfonyl halide of the formula III

$$X—SO_2NH—R^2 \qquad \text{(III)},$$

in which $R^2$ has the abovementioned meaning and X denotes chlorine, bromine or fluorine, in the presence of at least one equivalent, based on the ester used, of a base.

4. A 3-amino-2-sulfamoylalk-2-enoic acid ester of the formula IV

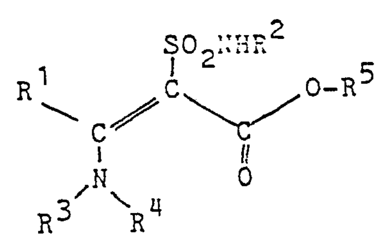

(IV)

in which

$R^1$ denotes a $C_1$-$C_{11}$ alkyl radical, a phenyl radical optionally substituted by halogen atoms or $C_1$-$C_4$ alkoxy, nitro or $C_1$-$C_2$ alkoxycarbonyl groups, the number of substituents on the phenyl radical being at most three, or a phenyl-$C_1$-$C_2$-alkyl radical in which the phenyl group can be substituted by one or two halogen atoms or $C_1$-$C_2$ alkoxy groups, and

$R^2$ denotes a hydrogen atom, a $C_1$-$C_{12}$ alkyl radical or a $C_6$-$C_{12}$ aryl radical which can be substituted by groups containing 0 to 3 heteroatoms and 0 to 2 carbon atoms,

$R^3$ and $R^4$ are identical or different and denote hydrogen, $C_1$-$C_4$ alkyl radicals which can also be linked together to form a ring, or $C_7$-$C_{12}$ aralkyl or $C_6$-$C_{12}$ aryl radicals having 0 to 2 heteroatoms, and $R^5$ denotes a branched or unbranched $C_1$-$C_{18}$ alkyl radical which can contain heteroatoms.

**Revendications**

1. Procédé de préparation d'oxo-2 sulfonamides répondant à la formule I :

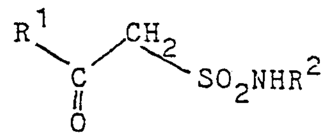

(I)

dans laquelle :

$R^1$ représente un radical alkyle en $C_1$-$C_{11}$, un radical phényle éventuellement porteur d'atomes d'halogènes ou de radicaux alcoxy en $C_1$-$C_4$, nitro ou alcoxycarbonyles en $C_2$ ou $C_3$, le nombre des substituants portés par le radical phényle étant d'au plus trois, ou un radical phénylalkyle dont le radical alkyle contient un ou deux atomes de carbone et dont le radical phényle peut porter un ou deux atomes d'halogènes ou radicaux alcoxy en $C_1$ ou $C_2$, et

$R^2$ représente un atome d'hydrogène, un radical alkyle en $C_1$-$C_{12}$ ou un radical aryle en $C_6$-$C_{12}$ qui peut porter des radicaux contenant de 0 à 3 hétéroatomes et de 0 à 2 atomes de carbone,
procédé caractérisé en ce qu'on fait réagir un ester d'acide amino-3 alcène-2 oïque répondant à la formule II :

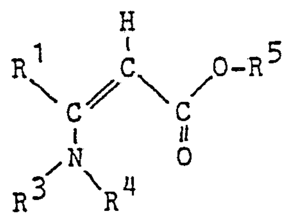

(II)

dans laquelle $R^1$ a la signification précédemment donnée, $R^3$ et $R^4$ représentent chacun, indépendamment l'un de l'autre, l'hydrogène, un radical alkyle en $C_1$-$C_4$, un radical aralkyle en $C_7$-$C_{12}$ ou aryle en $C_6$-$C_{12}$ contenant de 0 à 2 hétéroatomes, ou encore, alors qu'ils représentent chacun un alkyle, peuvent être unis l'un à l'autre et former ainsi un cycle, et $R^5$ représente un radical alkyle en $C_1$-$C_{18}$ linéaire ou ramifié, de préférence un radical alkyle en $C_1$-$C_4$, qui peut contenir des hétéroatomes, avec un halogénure d'amino-sulfonyle répondant à la formule III :

$$X—SO_2NH—R^2 \qquad (III)$$

dans laquelle $R^2$ a la signification précédemment donnée et X représente le chlore, le brome ou le fluor, en présence d'au moins un équivalent d'une base relativement à l'ester mis en jeu, et on soumet l'ester d'acide amino-3 sulfamoyl-2 alcène-2 oïque obtenu, qui répond à la formule IV :

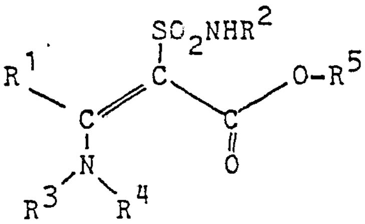

(IV)

dans laquelle $R^1$, $R^2$, $R^3$, $R^4$ et $R^5$ ont les significations précédemment données, à une saponification avec décarboxylation en milieu acide ou alcalin.

2. Oxo-2 sulfonamides répondant à la formule I :

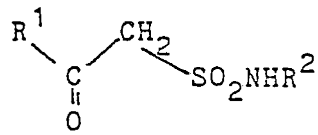

(I)

dans laquelle :

$R^1$ représente un radical alkyle en $C_1$-$C_{11}$, un radical phényle éventuellement porteur d'atomes d'halogènes ou de radicaux alcoxy en $C_1$-$C_4$ ou alcoxycarbonyles en $C_2$ ou $C_3$ (donc à alcoxy en $C_1$ ou $C_2$), le nombre des substituants portés par le radical phényle étant d'au plus trois, ou un radical phénylalkyle dont le radical alkyle contient un ou deux atomes de carbone et dont le radical phényle peut

porter un ou deux atomes d'halogènes ou radicaux alcoxy en $C_1$ ou $C_2$, et

$R^2$ représente un atome d'hydrogène, un radical alkyle en $C_1$-$C_{12}$ ou un radical aryle en $C_6$-$C_{12}$ qui peut porter des radicaux contenant de 0 à 3 hétéroatomes et de 0 à 2 atomes de carbone sauf ceux des composés de formule I dans lesquels

$R^1$ représente un alkyle en $C_1$-$C_4$ ou un phényle et $R^2$ l'hydrogène, et ceux dans lesquels

$R^1$ représente un phényle et $R^2$ un phényle, un cyclohexyle ou un n-butyle.

3. Procédé de préparation d'esters d'acides amino-3 sulfamoyl-2 alcène-2 oïques répondant à la formule IV :

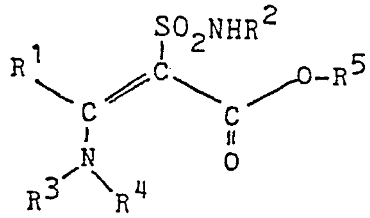

(IV)

dans laquelle

$R^1$ représente un radical alkyle en $C_1$-$C_{11}$, un radical phényle éventuellement porteur d'au plus trois substituants pris dans l'ensemble constitué par les atomes d'halogènes, les alcoxy en $C_1$-$C_4$, le nitro et les alcoxycarbonyles en $C_2$ ou en $C_3$, ou un radical phénylalkyle dont l'alkyle contient un ou deux atomes de carbone et dont le phényle peut porter un ou deux atomes d'halogènes ou radicaux alcoxy en $C_1$ ou $C_2$,

$R^2$ représente un atome d'hydrogène, un radical alkyle en $C_1$-$C_{12}$ ou un radical aryle en $C_6$-$C_{12}$ qui peut porter des radicaux contenant de 0 à 3 hétéroatomes et de 0 à 2 atomes de carbone,

$R^3$ et $R^4$ représentent chacun, indépendamment l'un de l'autre, l'hydrogène, un radical alkyle en $C_1$-$C_4$, un radical aralkyle en $C_7$-$C_{12}$ ou aryle en $C_6$-$C_{12}$ contenant de 0 à 2 hétéroatomes, ou encore, alors qu'ils désignent chacun un alkyle, sont unis l'un à l'autre et forment ainsi un cycle, et $R^5$ représente un radical alkyle en $C_1$-$C_{18}$, linéaire ou ramifié, qui peut contenir des hétéroatomes,

procédé caractérisé en ce qu'on fait réagir un acide amino-3 alcène-2 oïque répondant à la formule II :

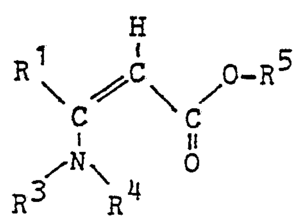

(II)

dans laquelle $R^1$, $R^3$, $R^4$ et $R^5$ ont les significations précédemment données, avec un halogénure d'amino-sulfonyle répondant à la formule III :

$$X—SO_2NH—R^2 \qquad (III)$$

dans laquelle $R^2$ a la signification précédemment donnée, et X représente le chlore, le brome ou le fluor, en présence d'au moins un équivalent d'une base par rapport à l'ester mis en jeu.

4. Esters d'acides amino-3 sulfamoyl-2 alcène-2 oïques répondant à la formule IV :

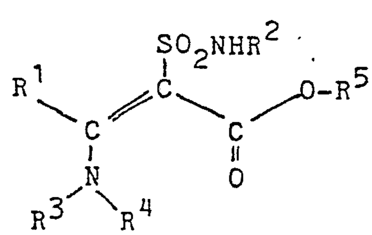

(IV)

dans laquelle

$R^1$ représente un radical alkyle en $C_1$-$C_{11}$, un radical phényle éventuellement porteur d'atomes d'halogènes ou de radicaux alcoxy en $C_1$-$C_4$, nitro ou alcoxycarbonyles en $C_2$ ou $C_3$, le nombre de substituants portés par le radical phényle étant d'au plus trois, ou un radical phénylalkyle dont l'alkyle contient un ou deux atomes de carbone et dont le phényle peut porter un ou deux atomes d'halogènes ou radicaux alcoxy en $C_1$ ou $C_2$,

$R^2$ représente un atome d'hydrogène, un radical alkyle en $C_1$-$C_{12}$ ou un radical aryle en $C_6$-$C_{12}$ qui

peut porter des radicaux contenant de 0 à 3 hétéroatomes et de 0 à 2 atomes de carbone,

$R^3$ et $R^4$ représentent chacun, indépendamment l'un de l'autre, l'hydrogène, un radical alkyle en $C_1$-$C_4$, ou un radical aralkyle en $C_7$-$C_{12}$ ou aryle en $C_6$-$C_{12}$ contenant de 0 à 2 hétéroatomes, $R^3$ et $R^4$, lorsqu'ils représentent des alkyles, pouvant également être unis l'un à l'autre et former ainsi un cycle, et $R^5$ représente un radical alkyle en $C_1$-$C_{18}$, ramifié ou non, qui peut contenir des hétéroatomes.